(19) Europäisches Patentamt
European Patent Office
Office européen des brevets

(11) Veröffentlichungsnummer: **0 099 960**
**B1**

# (12) EUROPÄISCHE PATENTSCHRIFT

(45) Veröffentlichungstag der Patentschrift: **10.04.85**

(51) Int. Cl.⁴: **C 07 C 101/20,** C 07 K 5/02

(21) Anmeldenummer: **83103376.6**

(22) Anmeldetag: **07.04.83**

(54) **Verfahren zur Herstellung von Niederalkylestern von N-L-alpha-Aspartyl-L-phenylalanin und neue Zwischenprodukte zu deren Herstellung.**

(30) Priorität: **20.07.82 CH 4433/82**

(43) Veröffentlichungstag der Anmeldung: **08.02.84 Patentblatt 84/6**

(45) Bekanntmachung des Hinweises auf die Patenterteilung: **10.04.85 Patentblatt 85/15**

(84) Benannte Vertragsstaaten: **AT BE CH DE FR GB IT LI LU NL SE**

(56) Entgegenhaltungen:
**EP - A - 0 035 047**
**EP - A - 0 048 345**

(73) Patentinhaber: **EPROVA AG Forschungsinstitut, Im Laternenacker 5, CH-8200 Schaffhausen (CH)**
(84) Benannte Vertragsstaaten: **BE CH DE GB IT LI LU NL SE AT**

(73) Patentinhaber: **AUTAPHARM S.A., Chenaleyres, CH 1782 Autafond (CH)**
(84) Benannte Vertragsstaaten: **FR**

(72) Erfinder: **Müller, Hans-Rudolf, Dr. sc. techn., Beckenwäldli 18, CH-8207 Schaffhausen (CH)**
Erfinder: **Bollinger, Heinrich, Dr.-Phil. II, Neuweg 11, CH-8222 Beringen (CH)**

(74) Vertreter: **Zutter, Hans Johann Niklaus, EPROVA AG Forschungsinstitut Im Laternenacker 5, CH-8200 Schaffhausen (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Niederalkylester von N-L-α-Aspartyl-L-phenylalanin, insbesondere der α-Aspartam genannte N-L-α-Aspartyl-L-phenylalanin-1-methylester, sind gesuchte kalorienarme, hochverträgliche Süssstoffe.

Zahlreiche Verfahren zur Herstellung von α-Aspartam und homologen Estern sind in der Literatur beschrieben. Diese sind entweder zu umständlich und daher für die Herstellung im technischen Massstab ungeeignet, oder sie sind nicht spezifisch und führen zu einem Gemisch von α- und β-Aspartam.

Gemäss den meisten technischen Verfahren zur Herstellung von α-Aspartam und Homologen wird ein am Stickstoffatom geschütztes oder sogar ungeschütztes L-Asparaginsäureanhydrid mit dem gewünschten L-Phenylalaninalkylester umgesetzt, etwa gemäss

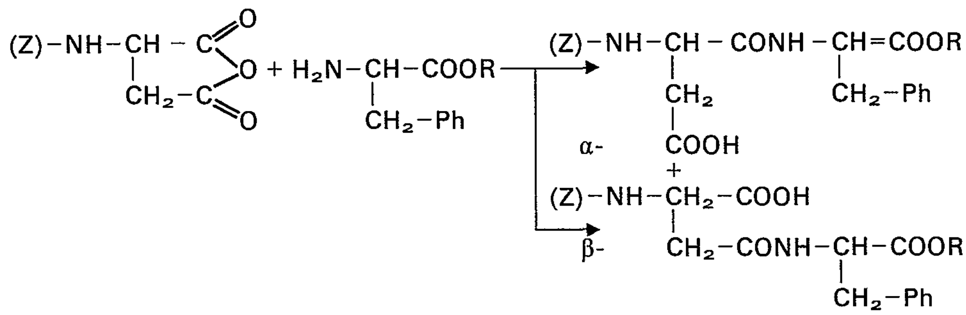

Nach der Abspaltung der Schutzgruppe wird eine Mischung der α-+β-Form des Produktes ers Produktes erhalten.

Versuchen, durch geeignete Reaktionsbedingungen das Mischungsverhältnis zugunsten von α-Aspartam zu beeinflussen, war bisher nur ein bescheidener Erfolg beschieden. Da nur die α-Form als Süssstoff brauchbar ist, wird die Ökonomie der Herstellung durch nicht unterdrückbare Bildung der β-Form dramatisch verschlechtert.

Selektive Methoden zur Herstellung von α-Aspartam und homologen Estern gehen etwa aus vom bekannten L-Asparaginsäure-N-carbonsäureanhydrid:

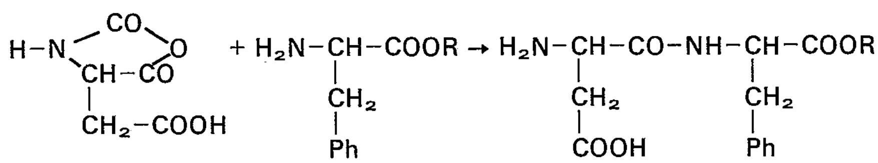

Dieses führt, wie das obige Reaktionsschema deutlich macht, selektiv zu α-Aspartam. Die Instabilität des genannten Anhydrids verunmöglicht jedoch bisher eine technische Anwendung dieses theoretisch einfachen Verfahrens.

Eine weitere selektive Methode wurde von Davey *et al.*, „J. Org. Chem. Soc." *1966* (5), 555-66 beschrieben. Gemäss dieser Methode wird α-Trichlorphenyl-β-benzyl-N-benzyloxycarbonyl-L-aspartat mit einem L-Phenylalaninester umgesetzt und erhaltenes Produkt anschliessend hydrogenolytisch gespalten.

Eine ähnliche Synthese basierend auf α-p-Nitrophenyl-β-benzyl-N-benzyloxycarbonyl-L-aspartat ist in der FR-PS Nr. 1577545 beschrieben.

Der entscheidende Nachteil der beiden, zuletzt genannten selektiven Methoden ist der hohe Aufwand für die Herstellung des N-Carbobenzoxygeschützten asymmetrischen Diesters von L-Asparaginsäure.

Verschiedene Autoren haben die Umsetzung von β-Benzyl-N-benzyloxycarbonyl-L-asparaginsäure mit L-Phenylalaninmethylester in Gegenwart von N,N'-Dicyclohexylcarbodiimid als Kondensationsmittel beschrieben. Diese führt selektiv zu β-Benzyl-N-benzyloxycarbonyl-L-aspartyl-L-phenylalaninester.

Die Methode fällt für eine technische Herstellung ausser Betracht, da sowohl das Ausgangsmaterial als auch das Kondensationsmittel zu kostspielig ist.

Kürzlich haben Vinick *et al.*, „Tetrahedron Letters" *23* (13), 1315-19 (1982), US-PS Nr. 4256897, ein selektives Verfahren beschrieben, das auf der Verwendung von L-Asparaginsäure-N-thiocarboxyanhydrid als Zwischenprodukt beruht. Dieses Verfahren hat den Nachteil massiver Geruchsbelästigung und ist daher für die Herstellung eines Nahrungsmittelbestandteils nicht besonders geeignet.

Es besteht nach wie vor ein erhebliches Bedürfnis für ein wirtschaftliches, selektives Verfahren zur Herstellung von α-Aspartam und homologen Estern.

Die vorliegende Erfindung füllt diese empfindliche Lücke. Sie beruht auf der neuen Kombination von zwei Massnahmen.

1. Der Verwendung einer einfach und selektiv entfernbaren Schutzgruppe an der β-Carboxyl-Funktion von L-Asparaginsäure.

2. Der Anwendung einer billigen und einfach

entfernbaren Schutzgruppe an der Amino-Funktion von L-Asparaginsäure.

Die erfindungsgemässe Methode ist dadurch gekennzeichnet, dass man den β-Benzylester oder einen substituierten β-Benzylester von L-Asparaginsäure als Ausgangsmaterial verwendet und eine 1-Alkyl-2-acylvinylgruppe, beispielsweise die 1-Methyl-2-acetylvinyl- oder 1-Methyl-2-ethoxycarbonylvinylgruppe, für den Schutz der Aminogruppe benutzt.

Glücklicherweise lassen sich die Ausgangsprodukte, die L-Asparaginsäure-4-(arylmethyl)ester, beispielsweise der L-Asparaginsäure-β-benzylester, mit relativ guter Ausbeute direkt durch Veresterung von L-Asparaginsäure mit Benzylalkohol oder einem substituierten Benzylalkohol in Gegenwart einer Säure herstellen.

Als Reagens für die Bildung der genannten Schutzgruppe an der Amino-Funktion von L-Asparaginsäure können beispielsweise die wohlfeilen Handelsprodukte Acetylaceton, Acetessigester oder Homologe davon verwendet werden. Gegenstand der Erfindung ist demnach ein neues selektives und wirtschaftliches Verfahren zur Herstellung von α-Aspartam und homologen Estern.

Das erfindungsgemässe Verfahren zur Herstellung von N-L-α-Aspartyl-L-phenylalanin-1-niederalkyester ist dadurch gekennzeichnet, dass man

a) einen L-Asparaginsäure-4-(arylmethyl)-ester mit einem 1,3-Diketon oder Acylessigester in Gegenwart einer Base umsetzt, das dabei gebildete Salz von L-N-(1-Alkyl-2-acylvinyl- oder 1-Alkyl-2-alkoxycarbonylvinyl)-asparaginsäure-4-(arylmethyl)ester mit einem organischen oder anorganischen Säurehalogenid der Formel X–A, worin X ein Halogenatom, Chlor oder Brom bedeutet, oder mit einem Säureanhydrid umsetzt und erhaltenes reaktives Anhydrid von L-N-(1-Alkyl-2-acylvinyl- oder 1-Alkyl-2-alkoxycarbonylvinyl)asparaginsäure-4-(arylmethyl)ester der Formel I

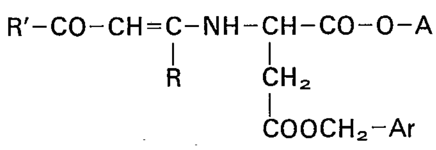

(I)

worin

R einen Alkylrest mit 1 bis 6 C-Atomen,

R′ einen Alkyl- oder Alkoxyrest mit 1 bis 6 C-Atomen

Ar den Phenylrest oder einen Nitro-, Halogen- oder Alkylphenylrest, und

A einen Acyl- oder Alkoxycarbonylrest mit 2 bis 12 C-Atomen oder den Rest einer Phosphor- oder Phosphorigsäure, Schwefelsäure, Schwefligsäure oder Sulfonsäure

bedeuten,

mit einem L-Phenylalaninniederalkylester umsetzt, oder

b) ein Salz von L-N-(1-Alkyl-2-acylvinyl- oder 1-Alkyl-2-alkoxycarbonylvinyl)asparaginsäure-4-arylmethylester mit einem Phosphorazo-L-phenylniederalkylester umsetzt und den nach a oder b erhaltenen N-L-α-N′-(1-Alkyl-2-acylvinyl- oder 1-Alkyl-2-alkoxycarbonylvinyl)aspartyl-L-phenylalanin-1-niederalkyl-4-(arylmethyl)-ester mit einer starken Säure behandelt, wobei die N-(1-Alkyl-2-acylvinyl- oder 1-Alkyl-2-alkoxycarbonylvinyl)-Schutzgruppe abgespalten wird, und den erhaltenen N-L-α-Aspartyl-L-phenylalanin-1-niederalkyl-4-(arylmethyl)ester einer katalytischen Hydrierung unterwirft, wobei die 4-(Arylmethyl)estergruppe selektiv gespalten und N-L-α-Aspartyl-L-phenylalanin-1-niederalkylester erhalten wird.

Gegenstand der Erfindung sind auch die neuen Zwischenprodukte, die N-L-α-N′-(1-Alkyl-2-acylvinyl- oder 1-Alkyl-2-alkoxycarbonylvinyl)-aspartyl-L-phenylalanin-1-niederalkyl-4-(arylmethyl)ester der Formel II

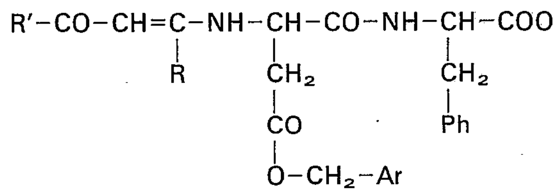

(II)

worin

R einen Alkylrest mit 1 bis 6 C-Atomen,

R′ einen Alkyl- oder Alkoxyrest mit 1 bis 6 C-Atomen,

Ar den Phenylrest oder einen Nitro-, Halogen- oder Alkylphenylrest, und

R″ den Methylrest oder einen Niederalkylrest mit 2 bis 4 C-Atomen

bedeuten.

Als Ausgangsstoffe für das Verfahren können der L-Asparingsäure-4-(phenylmethyl)ester oder ein substituierter (Phenylmethyl)ester verwendet werden, beispielsweise der (2-Nitrophenylmethyl)-, (3-Nitrophenylmethyl)- oder (4-Nitrophenylmethyl)ester, der (2-, 3- oder 4-Chlorphenylmethyl)ester, der (3,5-Dichlorphenylmethyl)- oder (2,4-Dichlorphenylmethyl)ester, der (2-, 3- oder 4-Tolylmethyl)ester, der (3,5-Dimethylphenylmethyl)ester oder der (2,4,6-Trimethylphenylmethyl)ester.

Als Reagens für die Bildung der Schutzgruppe an der Amino-Funktion werden gemäss der Erfindung ein 1,3-Diketon oder ein Acylessigsäureester verwendet. Wohlfeile Handelsprodukte wie Acetylaceton, Acetessigsäuremethyl-, -ethyl- und -isopropylester werden bevorzugt. In gleicher oder ähnlicher Weise lassen sich aber auch Propionylaceton, Butyrylaceton oder Propionylessigsäurealkylester verwenden. Durch Verwendung dieser weniger leicht zugänglichen Reagenzien werden indessen kaum Vorteile erzielt.

Als Basen für die Versalzung der α-Carboxyl-Funktion kommen in Betracht: Alkalihydroxide wie Natriumhydroxid, Kaliumhydroxid und Lithiumhydroxid oder tertiäre Amine wie Trimethylamin, Triethylamin, Tripropylamin, Tributylamin, Methyldiethylamin, Diethylpropylamin oder Dimethylisopropylamin, N-Methylpyrrolidin, N-Methylpiperidin und N-Methylmorpholin.

Als organische oder anorganische Säurehalogenide der Formel X–A und als Säureanhydride für die Bildung des gemischten Säureanhydrides der Formel I kommen in Betracht:

Kohlensäurehalbesterchloride wie Chlorameisensäuremethylester, -ethylester, -propylester-, isopropylester, -butylester, -isobutylester, -sek.-butylester, -t-butylester, -pentylester, -nitrophenylester, -chlorphenylester, -tolylester, -phenylmethylester, -phenylethylester.

Fettsäurehalogenide wie Acetylchlorid, Propionylbromid, 2-Methylpropionsäurechlorid, Buttersäurechlorid, Valeriansäurechlorid, Isovaleriansäurechlorid, Pivalinsäurechlorid, Pivalinsäurebromid.

Phosphor- und Phosphorigsäurehalogenide, zum Beispiel Phosphoroxychlorid, oder Chlorphosphite wie Ethyldichlorphosphit, Dimethylchlorphosphit, Diethylchlorphosphit, Ethylenchlorphosphit, o-Phenylenchlorphosphit.

Schwefel-, Schwefligsäure- und Sulfonsäurehalogenide wie Sulfurylchlorid, Thionylchlorid, Methansulfonsäurechlorid, Toluolsulfonsäurechlorid, Benzolsulfonsäurechlorid.

Säureanhydride wie $SO_3$, 2-Sulfobenzoesäureanhydrid, 3-Sulfopropionsäureanhydrid, Tetraethylpyrophosphit, Bis-1,2-phenylenpyrophosphit.

Als 1-Phenylalaninniederalkylester, welche mit dem gemischten Säureanhydrid der Formel I umgesetzt werden, kommen in Betracht: der L-Phenylalaninmethylester, ausserdem der Ethyl-, Propyl-, Isopropyl- oder Butylester.

Als starke Säure für die Abspaltung der N-(1-Alkyl-2-acylvinyl- oder 1-Alkyl-2-alkoxycarbonylvinyl)-Schutzgruppe kommen bevorzugt in Betracht: Salzsäure, Salpetersäure, Schwefelsäure, Phosphorsäure oder Ameisensäure.

*Beispiel 1:*

A) 22,3 g L-Asparaginsäure-4-(phenylmethyl)ester (0,1 mol) werden in 60 ml Benzylalkohol suspendiert und mit 10,4 ml Acetylaceton (0,1 mol) versetzt. Unter Rühren wird während 1 h eine Lösung von 6,59 g Kaliumhydroxid (85%ig) (0,1 mol) in 50 ml Benzylalkohol zugetropft, wobei die Temperatur auf 60 bis 65°C gehalten wird. Man rührt noch 2 h bei dieser Temperatur, kühlt danach auf Raumtemperatur ab und fällt das gebildete Produkt durch Zusatz von Diethylether oder isoliert es durch Einengen der Reaktionslösung im Vakuum und Aufnehmen des Rückstandes in Ethylacetat.

Nach dem Abfiltrieren und Trocknen erhält man 34,1 g Kaliumsalz von L-N-(1-Methyl-2-acetylvinyl)asparaginsäure-4-(phenylmethyl)ester, das sind 99% der Theorie.

Smp.: 101 bis 105°C unter Zersetzung

Äquivalentgewicht: berechnet 343,4; gefunden 348

B) 13,7 g Kaliumsalz von L-N-(1-Methyl-2-acetylvinyl)asparaginsäure-4-(phenylmethyl)ester (0,04 mol) werden in 100 ml Ethylacetat suspendiert und mit 0,55 ml Triethylamin versetzt. Die erhaltene Suspension wird bei −15°C mit 3,8 ml Chlorameisensäureethylester (0,04 mol) versetzt.

Es bildet sich das gemischte Anhydrid von L-N-(1-Methyl-2-acetylvinyl)asparaginsäure-4-(phenylmethyl)ester und Ethoxycarbonsäure. Nun fügt man unter Rühren bei −10°C eine Lösung von 0,04 mol L-Phenylalaninmethylester (hergestellt aus 8,6 g L-Phenylalaninmethylester · Hydrochlorid [0,04 mol] und 5,54 ml Triethylamin [0,04 mol] in 50 ml Ethylacetat) zur oben beschriebenen Reaktionslösung. Man rührt noch einige Stunden bei Raumtemperatur.

Die Reaktionslösung wird mit 20 ml 5N-Salzsäure versetzt und etwa 1 h heftig turbiniert. Nun werden die Phasen getrennt. Die Ethylacetatphase wird im Vakuum zur Trockene verdampft. Der ölige Rückstand wird mit verdünnter Salpetersäure verrieben, wobei Kristallisation eintritt. Das so erhaltene Produkt (15 g) besteht aus dem Nitrat von N-L-α-Aspartyl-L-phenylalanin-1-methyl-4-(phenylmethyl)ester.

Äquivalentgewicht: berechnet 447,45; gefunden 452,5

C) 5 g N-L-α-Aspartyl-L-phenylalanin-1-methyl-4-(phenylmethyl)ester · Nitrat (0,011 mol) werden in 75 ml Methanol enthaltend 6 ml 2N-Salzsäure in Gegenwart von 0,5 g 5%igem Palladium/Kohle-Katalysator bei Raumtemperatur hydriert.

Der Katalyst wird abfiltriert und das Filtrat zur Trockene verdampft. Der Rückstand wird mit natriumhydrogencarbonathaltigem Wasser verrieben.

Der gebildete N-L-α-Aspartyl-L-phenylalanin-1-methylester wird abfiltriert und getrocknet. Man erhält 2,9 g α-Aspartam, das sind 90% der Theorie, das nach Umkristallisieren des Wassers bei 246°C schmilzt.

*Beispiele 2 bis 8:*

In analoger Weise erhält man α-Aspartam, wenn man als Ausgangsmaterial benutzt:

2. L-Asparaginsäure-4-(2-anitrophenylmethyl)-ester
3. L-Asparaginsäure-4-(3-nitrophenylmethyl)-ester
4. L-Asparaginsäure-4-(4-nitrophenylmethyl)-ester
5. L-Asparaginsäure-4-(4-chlorphenylmethyl)-ester
6. L-Asparaginsäure-4-(2,4-chlorphenylmethyl)ester
7. L-Asparaginsäure-4-(4-tolylmethyl)ester
8. L-Asparaginsäure-4-(3,5-dimethylphenylmethyl)ester

*Beispiele 9 bis 23:*

Anstelle von Chlorameisensäureethylester im Beispiel 1B kann man auch verwenden:

9. Chlorameisensäuremethylester
10. Chlorameisensäurepropylester
11. Chlorameisensäureisobutylester
12. Chlorameisensäuresek.-butylester

13. Chlorameisensäurebenzylester
14. Chlorameisensäurephenylester
15. 2-Methylpropionsäurechlorid
16. Isovaleriansäurechlorid
17. Pivalinsäurebromid
18. Phosphoroxychlorid
19. Diethylchlorphosphit
20. Sulfonylchlorid
21. Methansulfonsäurechlorid
22. 2-Sulfobenzoesäureanhydrid
23. Tetraethylpyrophosphit

*Beispiel 24:*

A) 51,5 g Kaliumsalz von L-N-(1-Methyl-2-acetylvinyl)asparaginsäure-4-(phenylmethyl)-ester (0,15 mol) werden in 350 ml Ethylacetat suspendiert und mit 2 ml Triethylamin versetzt. In die erhaltene Suspension werden bei −15°C unter Rühren 18,1 g Pivaloylchlorid (0,15 mol) getropft.

Es bildet sich das gemischte Anhydrid von L-N-(1-Methyl-2-acetylvinyl)asparaginsäure-4-(phenylmethyl)ester und Pivalinsäure der Formel I, worin $A=(CH_3)_3C-CO-$ bedeutet.

Man rührt noch etwa 10 bis 30 min und lässt danach bei −10 bis −15°C innert 1 h eine Lösung von 0,15 mol L-Phenylalaninmethylester in 170 ml Ethylacetat zur Lösung des gemischten Anhydrides unter Rühren einlaufen. Man rührt noch einige Stunden bei −15°C. Die entstandene Suspension wird bei Raumtemperatur filtriert. Das Filtrat wird mit wässeriger Natriumhydrogencarbonatlösung und mit Wasser extrahiert.

Die erhaltene Ethylacetatlösung enthält N-L-α-N'-(1-Methyl-2-acetylvinyl)aspartyl-L-phenylalanin-1-methyl-4-(phenylmethyl)ester.

Dünnschichtchromatogramm (DC) auf Kieselgel, mit Laufmittel Toluol/Ethylacetat/Isopropanol/2N-Essigsäure=2:7:7:4; $R_f$=0,83

B) Die nach A erhaltene Lösung wird unter Rühren während 1 bis 1½ h mit 150 ml 2N-Salzsäure behandelt, wobei die Schutzgruppe abgespalten wird. Die wässerige Phase wird abgetrennt. Die organische Ethylacetatphase wird getrocknet und vollständig eingedampft. Aus dem Destillat können Acetylaceton und Ethylacetat zurückgewonnen werden.

Der Eindampfrückstand (64,3 g, das sind 101,8% der Theorie) besteht aus dem Hydrochloridadditionssalz von N-L-α-Aspartyl-L-phenylalanin-1-methyl-4-(phenylmethyl)ester.

Smp. (nach Umkristallisation aus Ethylacetat): 160-165°C

$[\alpha]_D^{20}=-6{,}0°$ (c=4% in 15N-Ameisensäure)

DC auf Kieselgel: Laufmittel Acetonitril/Dioxan/Wasser=9:1:4; $R_f$=0,63; Flecken mit Ninhydrin entwickelt

C) 50 g N-L-α-Aspartyl-L-phenylalanin-1-methyl-4-(phenylmethyl)ester · Hydrochlorid (0,119 mol) werden in 300 ml Methanol in Gegenwart von 1 g 5%igem Palladium/Kohle-Katalysator bei Raumtemperatur und bei Normaldruck hydriert.

Nachdem die berechnete Menge Wasserstoff verbraucht ist, kommt die Hydrierung zum Stillstand. Der Katalysator wird abfiltriert und das Filtrat eingeengt. Der Rückstand wird mit 400 ml Wasser verrührt und durch Zusatz von 2N-Natronlauge auf pH 4,2 gestellt. Das ausgeschiedene N-L-α-Aspartyl-L-phenylalanin-1-methylester · Hydrat (33,5 g, das sind 90% der Theorie) schmilzt bei 245-247°C.

$[\alpha]_D^{20}=+15°$ (c=4% in 15N-Ameisensäure)

DC auf Kieselgel mit Laufmittel Acetonitril/Dioxan/Wasser=9:1:4; $R_f$=0,4; Flecken mit Ninhydrin entwickelt

Äquivalentgewicht: berechnet 312,33; gefunden 315,36.

*Beispiele 25 bis 31:*

In analoger Weise, wie im Beispiel 1 beschrieben, erhält man α-Aspartam, wenn man Acetylaceton ersetzt durch

25. Acetessigsäureethylester
26. Acetessigsäuremethylester
27. Acetessigsäureisopropylester
28. Propionylessigsäureethylester
29. Propionylessigsäureisopropylester
30. Propionylaceton
31. Butyrylaceton

*Beispiel 32:*

A) 10,75 g L-Phenylalaninmethylester (0,06 mol) werden in 160 ml Dioxan und 10,7 g Pyridin bei ~10°C unter Rühren mit 4,1 g Phosphortrichlorid (0,03 mol) versetzt. Die gebildete Phosphorazoverbindung (Houben-Weyl's „Methoden der organischen Chemie", Bd. *15/2*, 237-240 [1974]) fällt aus. Man rührt noch 1 bis 3 h bei Raumtemperatur, setzt danach 20,6 g Kaliumsalz von L-N-(1-Methyl-2-acetylvinyl)asparaginsäure-4-(phenylmethyl)ester (0,06 mol) zu und rührt die Reaktionslösung zunächst bei Raumtemperatur und später während 5 h bei Rückflusstemperatur.

Nach dem Abkühlen wird die Reaktionslösung filtriert und das Filtrat zur Trockene verdampft. Der Eindampfrückstand (20 g) besteht gemäss Dünnschicht- und Liquidchromatogramm zur Hauptsache aus N-L-α-Aspartyl-L-phenylalanin-1-methyl-4-(phenylmethyl)ester.

B) 10 g des Rückstandes werden analog Beispiel 24C einer Hydrogenolyse unterworfen, wobei α-Aspartam erhalten wird.

## Patentansprüche

1. Verfahren zur selektiven Herstellung von N-L-α-Aspartyl-L-phenylalanin-1-niederalkylester, dadurch gekennzeichnet, dass man

a) einen L-Asparaginsäure-4-(arylmethyl)-ester mit einem 1,3-Diketon oder Acylessigester in Gegenwart einer Base umsetzt, das dabei gebildete Salz von L-N-(1-Alkyl-2-acylvinyl- oder 1-Alkyl-2-alkoxycarbonylvinyl)asparaginsäure-4-(arylmethyl)ester mit einem organischen oder anorganischen Säurehalogenid der Formel X−A, worin X ein Halogenatom, Chlor oder Brom bedeutet, oder mit einem Säureanhydrid umsetzt und erhaltenes reaktives Anhydrid von L-N-(1-Alkyl-2-acylvinyl- oder 1-Alkyl-2-alkoxycarbonylvinyl)asparaginsäure-4-(arylmethyl)ester der Formel (I)

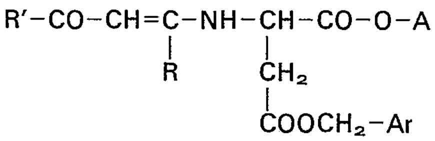

(I)

worin

R einen Alkylrest mit 1 bis 6 C-Atomen,

R′ einen Alkyl- oder Alkoxyrest mit 1 bis 6 C-Atomen,

Ar den Phenylrest oder einen Nitro-, Halogen- oder Alkylphenylrest, und

A einen Acyl- oder Alkoxycarbonylrest mit 2 bis 12 C-Atomen oder den Rest einer Phosphor- oder Phosphorigsäure, Schwefelsäure, Schwefligsäure oder Sulfonsäure

bedeuten,

mit einem L-Phenylalaninniederalkylester umsetzt, oder

b) ein Salz von L-N-(1-Alkyl-2-acylvinyl- oder 1-Alkyl-2-alkoxycarbonylvinyl)asparaginsäure-4-(arylmethyl)ester mit einem Phosphorazo-L-phenylniederalkylester umsetzt

und den nach a oder b erhaltenen N-L-α-N′-(1-Alkyl-2-acylvinyl- oder 1-Alkyl-2-alkoxycarbonylvinyl)aspartyl-L-phenylalanin-1-niederalkyl-4-(arylmethyl)ester mit einer starken Säure behandelt, wobei die N-(1-Alkyl-2-acylvinyl- oder 1-Alkyl-2-alkoxycarbonylvinyl)-Schutzgruppe abgespalten wird, und den erhaltenen N-L-α-Aspartyl-L-phenylalanin-1-niederalkyl-4-(arylmethyl)ester einer katalytischen Hydrierung unterwirft, wobei die 4-(Arylmethyl)estergruppe selektiv gespalten und N-L-α-Aspartyl-L-phenylalanin-1-niederalkylester erhalten wird.

2. N-L-α-N′-(1-Alkyl-2-acylvinyl- oder 1-Alkyl-2-alkoxycarbonylvinyl)aspartyl-L-phenylalanin-1-niederalkyl-4-(arylmethyl)ester der Formel (II)

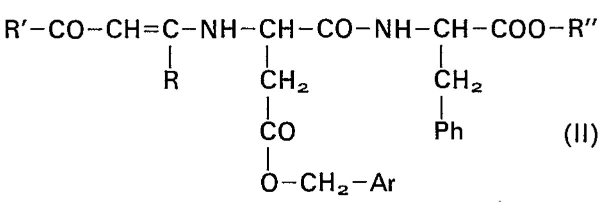

(II)

worin

R einen Alkylrest mit 1 bis 6 C-Atomen,

R′ einen Alkyl- oder Alkoxyrest mit 1 bis 6 C-Atomen,

Ar den Phenylrest oder einen Nitro-, Halogen- oder Alkylphenylrest, und

R″ den Methylrest oder einen Niederalkylrest mit 2 bis 4 C-Atomen

bedeuten.

## Claims

1. Process for the selective preparation of N-L-α-aspartyl-L-phenylalanine-1-lower alkyl ester, characterised in that

(*a*) an L-aspartic acid-4-(aryl methyl) ester is reacted with a 1,3-diketone or acyl acetic ester in the presence of a base, the salt formed thereby of L-N-(1-alkyl-2-acyl-vinyl- or 1-alkyl-2-alkoxycarbonyl vinyl)-aspartic acid-4-(aryl methyl) ester is reacted with an organic or inorganic acid halide of the formula X−A, wherein X signifies a halogen atom, chlorine or bromine, or with an acid anhydride, and resulting reactive anhydride of L-N-(1-alkyl-2-acyl-vinyl- or 1-alkyl-2-alkoxycarbonyl vinyl)-aspartic acid-4-(aryl methyl) ester of Formula (I)

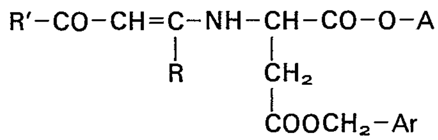

(I)

wherein

R is an alkyl group with 1 to 6 carbon atoms,

R′ is an alkyl or alkoxy group with 1 to 6 carbon atoms,

Ar signifies the phenyl group or a nitro- halo- or alkyl-phenyl group, and

A an acyl or alkoxycarbonyl group with 2 to 12 carbon atoms, or a phosphoric or phosphorous acid, sulphuric acid, sulphurous acid or sulphonic acid group,

is reacted with an L-phenylalanine-lower alkyl ester, or

(*b*) a salt of L-N-(1-alkyl-2-acyl-vinyl- or 1-alkyl-2-alkoxycarbonyl vinyl)-aspartic acid-4-(aryl methyl) ester is reacted with a phosphorazo-L-phenyl-lower alkyl ester,

and the N-L-α-N′-(1-alkyl-2-acyl-vinyl- or 1-alkyl-2-alkoxycarbonyl vinyl)-aspartyl-L-phenylalanine-1-lower alkyl-4-(aryl methyl) ester obtained in accordance with (*a*) or (*b*) is treated with a strong acid, the N-(1-alkyl-2-acyl-vinyl- or 1-alkyl-2-alkoxycarbonyl vinyl) protective group being thus split off, and the obtained N-L-α-aspartyl-L-phenylalanine-1-lower alkyl-4-(aryl methyl) ester is subjected to a catalytic hydrolysis wherein the 4-(aryl methyl) ester group is selectively split and N-L-α-aspartyl-L-phenylalanine-1-lower alkyl ester is obtained.

2. N-L-α-N′-(1-alkyl-2-acyl-vinyl- or 1-alkyl-2-alkoxycarbonyl vinyl)-aspartyl-L-phenylalanine-1-lower alkyl-4-(aryl methyl) ester of Formula (II)

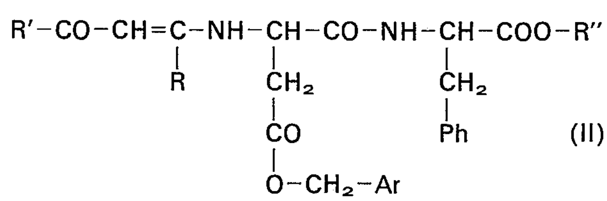

(II)

wherein

R signifies an alkyl group with 1 to 6 carbon atoms,

R′ an alkyl or alkoxy group with 1 to 6 carbon atoms,

Ar the phenyl group or a nitro- halo- or alkylphenyl group, and

R'' the methyl group or a lower alkyl group with 2 to 4 carbon atoms.

## Revendications

1. Procédé pour la préparation sélective des esters 1-alkyliques inférieurs de la N-L-α-aspartyl-L-phénylalanine, caractérisé en ce que:

a) on fait réagir un ester 4-arylméthylique de l'acide L-aspartique avec une 1,3-dicétone ou un ester acylacétique en présence d'une base, ce qui donne un sel d'ester 4-arylméthylique d'acide L-N-(1-alkyl-2-acylvinyl- ou 1-alkyl-2-alcoxycarbonylvinyl)aspartique qu'on fait réagir avec un halogénure d'acide organique ou minéral de formule X−A, dans laquelle X représente un atome d'halogène, de chlore ou de brome, ou avec un anhydride, ce qui donne un anhydride réactif de l'ester 4-arylméthylique de l'acide L-N-(1-alkyl-2-acylvinyl- ou 1-alkyl-2-alcoxycarbonylvinyl)-aspartique de formule (I)

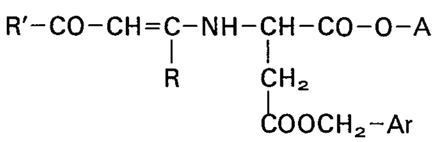

(I)

dans laquelle

R représente un groupe alkyle en C1-C6,

R' représente un groupe alkyle ou alcoxy en C1-C6,

Ar représente le reste phényle ou un reste nitro-, halogéno- ou alkylphényle, et

A représente un reste acyle ou alcoxycarbonyle contenant 2 à 12 atomes de carbone ou le reste d'un acide phosphorique ou phosphoreux, d'un acide sulfurique, d'un acide sulfureux ou d'un acide sulfonique,

qu'on fait réagir avec un ester alkylique inférieur de la L-phénylalanine, ou bien

b) on fait réagir un sel d'ester 4-arylméthylique d'acide L-N-(1-alkyl-2-acylvinyl ou 1-alkyl-2-alcoxycarbonylvinyl)aspartique avec un ester phosphorazo-L-phénylalkylique inférieur, et on traite l'ester 1-alkylique inférieur, 4-arylméthylique de la N-L-α-N'-(1-alkyl-2-acylvinyl- ou 1-alkyl-2-alcoxycarbonylvinyl)aspartyl-L-phénylalanine obtenu en a ou b par un acide fort qui provoque l'élimination du groupe protecteur N-(1-alkyl-2-acylvinyle ou 1-alkyl-2-alcoxycarbonylvinyle), et on soumet l'ester 1-alkylique inférieur, 4-arylméthylique de la N-L-α-aspartyl-L-phénylalanine obtenu à une hydrogénation catalytique qui provoque l'élimination sélective du groupe ester 4-arylméthylique et donne l'ester 1-alkylique inférieur de la N-L-α-aspartyl-L-phénylalanine.

2. Esters 1-alkyliques inférieurs, 4-arylméthyliques de N-L-α-N'-(1-alkyl-2-acylvinyl- ou 1-alkyl-2-alcoxycarbonylvinyl)aspartyl-L-phénylalanines de formule (II)

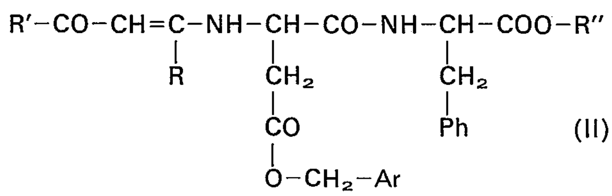

(II)

dans laquelle

R représente un groupe alkyle en C1-C6,

R' représente un groupe alkyle ou alcoxy en C1-C6,

Ar représente le reste phényle ou un reste nitro-, halogéno- ou alkylphényle, et

R'' représente le reste méthyle ou un groupe alkyle inférieur en C2-C4.